Europäisches Patentamt

European Patent Office

Office européen des brevets

(11) Publication number: **0 222 717**
**A2**

# (12) EUROPEAN PATENT APPLICATION

(21) Application number: **86850348.3**

(22) Date of filing: **13.10.86**

(51) Int. Cl.⁴: **A 61 L 31/00**
**A 61 L 27/00**

(30) Priority: **01.11.85 SE 8505158**

(43) Date of publication of application:
**20.05.87 Bulletin 87/21**

(84) Designated Contracting States:
**CH DE FR GB LI SE**

(71) Applicant: **The Institute for Applied Biotechnology**
**Box 33053**
**S-400 33 Göteborg (SE)**

(72) Inventor: **Branemark, Per-Ingvar**
**Ändergatan 3**
**S-431 39 Mölndal (SE)**

**Erlandsson, Björn-Erik**
**Dr. Forselius gata 2**
**S-413 26 Göteborg (SE)**

**Bjursten, Lars-Magnus**
**Linnegatan 9**
**S-413 04 Göteborg (SE)**

**Kasemo, Bengt**
**Tjärkil 1400**
**S-644 00 Mellerud (SE)**

(74) Representative: **Olsson, Gunnar**
**Nobel Corporate Services Patents and Trademarks**
**S-691 84 Karlskoga (SE)**

(54) Implant material.

(57) A material of unallyed titanium for implantation in body tissue wherein the surface inorganic atomic layer of the material contains

Ti 29 - 33
O 61 - 67

expressed in atomic per cent. In addition, the surface layer may not include amounts of other atoms about certain maximums. By using in living tissue it has been found that such a surface composition has given particularly positive tissue reactions.

**Description**

IMPLANT MATERIAL

The present invention relates to a material of titanium for implantation in body tissue.

Artificial materials in the form of implants have long been used in the human body for the purpose of reconstructing congenital or acquired defects in structure and function.

As an example of this, mention may be made of the use of metal elements in the form of spikes, screws, plates etc for fixing of bone fractures so that healing is expedited. In orthopaedics, such metal elements have long been used with relatively good success.

For purposes other than fixation of bone fractures etc it has, however proved difficult to attain the desired clinical results, particularly in situations where the artificial material is intended to function for a long period of time, in most cases throughout the life of the patient.

A distinguishing feature of those cases in which a good result has not been attained is that sufficiently close contact between organized living tissues for example bone tissue, and artificial material has not been attained, for example in order to permit an adequate transfer of force between tissue and implant.

Close contact between tissue and implant is important, for instance, also in those cases where the implant is arranged to penetrate skin, mucosa, blood vessels, intestines or other natural barriers. In this way, the onset of infection, inflammation and rejection through invasion of bacteria, secretion and other undesirable substances is prevented.

If good clinical results are actually to be attained with implant materials, particularly in such contexts where exacting demands are imposed on the establishment of a close contact between the implant material and the living tissue, both a refined surgical technique and an appropriate elaboration of the implant are required, in the case of the latter, both with regards to its physical and chemical properties and to its geometrical form.

One field in which the most progress has been made insofar as good clinical results are obtained are jaw boneanchored dental bridges, the anchorage of which is based on the use of anchoring elements of unalloyed titanium. Here the osseointegration principle developed by professor Brånemark and his colleagues has been used clinically with good results for more than 20 years and described in, for instance:

P-I Brånemark et al, "Osseointegrated titanium fixtures in the treatment of edentuolousness biomaterials" January, 1983, Vol. 4, and R. Skalak "Biomechanical considerations in osseointegrated prosthese" The Journal of prosthetic Dentistry, June 1983, Volume 49, Number 6.

Numerous factors have been found to be essential for the good clinical result. A refined surgical technique is, as mentioned heretofor, important for the good result, but also e g implant material and particularly the structure and composition of the surface layer of the implant is very important.

In order to achieve good bonding of the implant into body tissue it is essential to reduce negative biological reactions to a minimum and aim at obtaining, predominantly positive reactions to be induced. The atomic composition of the surface layer in this contex of decisive importance for its reactions on account of the fact that most interactions between the implant material and the surrounding body tissue are confined to an area in the immediate vicinity of the surface layer. The weakest type of interaction here is the so-called van der Waal's forces. A somewhat more powerful bond is established between permanent dipoles of the biomolecules and of the implant surface layer. Hydrogen bond also plays an important role and is roughly equally as strong as the dipolar bonds. But the strongest bond obtained here is, however, through covalent ionic bonding. These are severely affected by the surface-conditions at atomic level of the implant material, i e it is strongly material specific. Certain local defects and impurities in the surface may also display strong bonds, but imply at the same time a tangible risk of unwanted immunological effects.

It is, however, not only the strength of the bonds but also their permanence and the change in the conformation of the biomolecules that are important. The permanence of a specific bond can be predicted through the bonding energy. At body temperature, most covalent and ionic bonds are largely reversible, whereas the weaker types of bond may have a life of only a second or so. The type of bond, therefore, determines if the interface between implant and body tissue is to be regarded as static or dynamic. It is known that a change in the conformation of biomolecules can lead to unwanted effects of an immunological nature, such as induction of allergic reactions or initiation of inflammatory reactions.

It is important to stress that primary reactions that take place at the surface may, in turn, induce secondary reactions in the biosystem, for example through deposition of impurities and corrosion products, and through desorption of denatured proteins etc.

An important partial effect for the biocompatability of a titanium implant is its influence on the activation of inflammatory signal systems and on the occurrence of denaturing or conformation changes of biomolecules on its surface. This latter effect may give rise to allergic rejection reactions. Both effects lead to deteriorated implant function and can jeopardize the retention of the implant in the body. The effects can be checked, for example, by the method described in Swedish patent application No. 84.02653-3, whereby an analysis is performed of the activation by the surface of a complement system, a signal system for releasing of, among other things, rejection reactions.

The object of the present invention is to provide an implant material of unalloyed titanium the surface atomic condition of which is such that healing of the implant is optimized and that the bonding of the biomolecules to the surface is strengthened without the aforesaid unwanted effects occurring.

The invention is substantially characterized in that in the surface the inorganic atomic layer of the material contains:

Ti 29 - 33
O 61 - 67

expressed in atomic per cent.

It quite surprisingly has been found by using titanium implants in living tissue that such a surface composition has given particularly positive tissue reactions.

The atomic ratio between titanium and the oxygen is thus roughly one to two. This shows that the oxygen present in the surface as earlier assumed is largely bonded as titanium dioxide but according to the present invention the atomic proportions of titanium and oxygen are very critical and should not diverge outside the defined limits. The surface layer in question is generally about 25 angstroms (Å) thick.

Further, it is important for the present invention that the surface layer besides titanium and oxygen also displays the following maximum atomic proportions of the elements:

Ca <2.0
Na <4.0
Zn <2.0

and other elements <0.5 each expressed in atomic per cent of the inorganic layer.

The surface layer of the titanium implant can thus display small concentrations of Ca, Na and Zn which, however, are nevertheless so small that they have not revealed any discernible disruptive effect on the bonding of the biomolecules to the implant material.

In addition to the above elements, an absorbed organic surface layer containing carbon and nitrogen with the following proportions expressed in atomic per cent are found according to the invention:

N <8
C <50

The relatively high concentration of carbon is shown by ESCA to reflect the presence of hydrocarbons on the surface. This may have a favourable effect on the bonding of the biomolecules to the implant since reactive groups in the surface layer which are formed upon processing of the material are thus blocked and denaturing and conformation changes of the biomolecules are prevented.

The surface composition described heretofore with the specified atomic proportions has particularly well been found to satisfy requirements to achieve very good tissue integration. In the light of what has been said above about the potential sensitivity of the biosystem to even small surface impurities, it is a requirement that the specified proportions be satisfied in order for tissue integration to be guaranteeable.

In the following a description will be given of how the implant material can be prepared and the results of some different surface analysis (e g ESCA analysis) of the implants presented.

As mentioned heretofor, unalloyed titanium material displays very good healing in body tissue. A condition for this is, however, that titanium material is handled in a correct manner, all the way from processing procedure, cleaning and storage to sterilization and other treatment at the installation operation.

The machining of the material takes place with chipforming tools with special demands on cutting speed etc. Exacting demands are imposed on the cutting oil. The titanium material is machined by scoring the surface with a cutting rate of less than 20 m/min under a cooling with a paraffinic oil. The coolant employed should not be e g an aromatic mineral oil and/or should not contain sulfur. In addition, in order to obtain the surface layer required by the present invention, it is necessary to clean the titanium material very carefully.

An example of how this cleaning can take place for an implant material which reveals a surface condition in accordance with the invention is the following:

The titanium components are subjected to a number of cleaning baths in titanium vessels. The cleaning is followed by heat sterilization.

The procedure has the requirement that reactive groups arising as a result of the machining are blocked in a carefully controlled environment which may consists of a liquid, for instance cutting oil, butanol, ethanol, etc., or of a gas phase, for example alcohol or hydrocarbon.

An analysis of five implants, which have proved to give particularly good results and are produced as above have given the following atomic proportions of their surfaces:

| Element | 1. | 2. | 3. | 4. |
|---------|------|------|------|------|
| Ti | 29.9 | 31.1 | 31.0 | 32.3 |
| O | 63.6 | 65.2 | 64.3 | 62.5 |

3

| | | | | |
|---|---|---|---|---|
| Ca | 1.5 | 1.9 | 1.8 | 1.5 |
| Na | 3.1 | 0.7 | 1.8 | 2.3 |
| Cu | 0.2 | – | 0.2 | 0.2 |
| Zn | 1.1 | 0.9 | 0.9 | 1.1 |
| N | 4.2 | 4.5 | 3.8 | 6.9 |
| C | 28.1 | 32.9 | 39.8 | 39.2 |

**Oxide thickness:** 26 A    28.5 A    25 A    26 A

All concentrations are stated in atomic per cent. Carbon and nitrogen are assumed to be present in the form of an absorbed organic surface layer. The presence of this layer has not been counted as the concentrations for other elements have been specified.

In other words, the percentages of nitrogen and carbon are atomic per cents based upon the total amount of the surface layer including the adsorbed organic layer containing carbon and nitrogen. The atomic per cents given for the Ti, O, Ca, Na and Zn, and any other element (as eg Cu) are the relative amounts present in the inorganic layer, per se, substantially containing the titanium and oxygen atoms and not including the adsorbed organic layer.

The minimum detection level of the equipment employed is about 0.5 atomic per cent.

Titanium implants with the specified surface composition may be checked according to the method described in the aforesaid Swedish patent application No. 84.02653-3. Hereby they reveal a homogeneous and low activation of the complement system, without any patches with high activation.


**Claims**

1. A material of unalloyed titanium for implantation in body tissue, **characterized in that** in the surface the inorganic atomic layer of the material contains:
Ti 29 __ 33
O 61 - 67
expressed in atomic per cent.

2. The material of Claim 1 wherein said layer is about 25 angstroms thick.

3. A material according to Claim 1, **characterized in that** the layer besides titanium and oxygen also contains the following maximum proportions of the elements:
Ca < 2.0
Na < 4.0
Zn < 2.0
and other elements < 0.5 each
expressed in atomic per cent.

4. A material according to Claim 1, **characterized in that** the inorganic atomic layer reveals an absorbed organic surface layer which contains the following proportions:
N < 8
C < 50
expressed in atomic per cent.